(19) Europäisches Patentamt
European Patent Office
Office européen des brevets

(11) Veröffentlichungsnummer : **0 152 556**
**B1**

(12) # EUROPÄISCHE PATENTSCHRIFT

(45) Veröffentlichungstag der Patentschrift : **21.01.87**

(21) Anmeldenummer : **84114255.7**

(22) Anmeldetag : **26.11.84**

(51) Int. Cl.[4] : **C 07 D333/22**, C 07 D307/46, C 07 D213/50, C 07 D207/32, C 07 D231/12, A 61 K 31/38, A 61 K 31/34, A 61 K 31/44, A 61 K 31/40, A 61 K 31/41// C07C93/06, C07D409/12

(54) **Aminopropanolderivate von substituierten 2-Hydroxy-propiophenonen, Verfahren zu ihrer Herstellung und diese enthaltende therapeutische Mittel.**

(30) Priorität : **02.12.83 DE 3343671**

(43) Veröffentlichungstag der Anmeldung : **28.08.85 Patentblatt 85/35**

(45) Bekanntmachung des Hinweises auf die Patenterteilung : **21.01.87 Patentblatt 87/04**

(84) Benannte Vertragsstaaten : **AT BE CH DE FR GB IT LI NL SE**

(56) Entgegenhaltungen :
**EP-A- 0 008 108**
**EP-A- 0 075 207**
**FR-A- 2 400 515**
**CHEMICAL ABSTRACTS, Band 82, Nr. 25, 23. Juni 1975, Seite 515, Nr. 170660a, COLUMBUS, OHIO, (US)**

(73) Patentinhaber : **BASF Aktiengesellschaft**
***Carl-Bosch-Strasse 38***
**D-6700 Ludwigshafen (DE)**

(72) Erfinder : **Franke, Albrecht, Dr.**
**Mandelring 11**
**D-6706 Wachenheim (DE)**
Erfinder : **Spiegler, Wolfgang, Dr.**
**Westpreussenstrasse 5**
**D-6520 Worms (DE)**
Erfinder : **Siegel, Hardo, Dr.**
**Hans-Purrmann-Allee 25**
**D-6720 Speyer (DE)**
Erfinder : **Mueller, Claus D. Dr.**
**Im Schaflaeger 30**
**D-6806 Viernheim (DE)**
Erfinder : **von Philipsborn, Gerda, Dr.**
**Naechstenbacher Weg 35**
**D-6940 Weinheim (DE)**
Erfinder : **Lenke, Dieter, Prof. Dr.**
**Kekuléplatz 1**
**D-6700 Ludwigshafen (DE)**
Erfinder : **Gries, Josef, Dr.**
**Roemerweg 43**
**D-6706 Wachenheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 152 556 B1

**Beschreibung**

Gegenstand der Erfindung sind neue Aminopropanolderivate von β-substituierten 2-Hydroxy-propiophenonen, ihr Herstellungsverfahren sowie diese enthaltende therapeutische Mittel, die als Antiarrhythmika verwendet werden können.

Aus DE-OS 20 01 431, DE-OS 31 33 814 und DE-OS 32 26 863 ist bekannt, daß die Aminopropanolderivate des 2-Hydroxy-β-phenyl-propiophenons antiarrhythmisch wirksam sind. Das gilt besonders für das 2-(2'-Hydroxy-3'-n-propylamino-propoxy)-β-phenyl-propiophenon-hydrochlorid, das unter der Bezeichnung Propafenon als Antiarrhythmikum bekannt ist. Aufgabe der Erfindung ist es, demgegenüber hinsichtlich Wirkstärke nach oraler Applikation verbesserte Antiarrhythmika zur Verfügung zu stellen.

Es würde nun gefunden, daß Aminopropanolderivate der Formel I

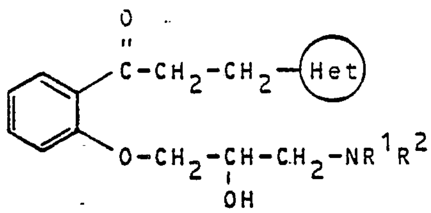

(I)

in der

$R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu insgesamt 9 C-Atomen, oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5- bis 7-gliedrigen gesättigten heterocyclischen Ring bilden, der durch einen oder zwei Phenyl- und/oder Hydroxyreste substituiert sein und ein Sauerstoff- oder Stickstoffatom als weiteres Heteroatom im Ring enthalten kann, wobei ein solches zusätzliches Stickstoffatom durch einen Alkylrest mit 1 bis 3 C-Atomen oder einen Phenylrest substituiert sein kann, und

—(Het) die Reste 2-Thienyl, 3-Thienyl, 2-Pyridyl, 3-Pyridyl, 4-Pyridyl, 2-Furyl, 2-(1-Alkyl)-pyrryl, 3-(1-Alkyl)-pyrryl und 4-(1-Alkyl)-pyrazolyl bedeutet, wobei der Alkylrest 1 bis 3 Kohlenstoffatome enthält, und ihre physiologisch verträglichen Säureadditionssalze wertvolle pharmakologische Eigenschaften aufweisen.

Von den Verbindungen der Formel I sind diejenigen besonders hervorzuheben, bei denen die Gruppe $NR^1R^2$ den Piperidin-, Piperazin-, N-Methyl-piperazin-, Morpholin- oder Diisopropylaminorest bedeutet, sowie solche, bei denen $R^1$ und/oder $R^2$ Wasserstoff, den Propyl-, Butyl-, Pentyl-, Alkoxyalkyl und Hydroxyalkylrest bedeutet, wobei n-Propyl, Isopropyl, n-Butyl, Isobutyl, tert.-Butyl, n-Pentyl, sec.-Pentyl, Isopentyl, Neopentyl, β-Methoxyethyl und β-Hydroxyethyl genannt seien. Besonders bevorzugte heterocyclische Reste sind der Pyridyl-, der 2-(1-Methyl)-pyrryl-, 3-(1-Methyl)-pyrryl und der 4-(1-Methyl)-pyrazolyl-Rest.

Außer den in den Beispielen angegebenen Verbindungen seien beispielsweise genannt :

2-(2'-Hydroxy-3'-isopropylamino-propoxy)-β-(2-thienyl)-propiophenon
2-(2'-Hydroxy-3'-n-pentylamino-propoxy)-β-(2-thienyl)-propiophenon
2-[2'-Hydroxy-3'-(2-methoxyethylamino)-propoxy]-β-(2-thienyl)-propiophenon
2-(2'-Hydroxy-3'-n-propylamino-propoxy)-β-(3-thienyl)-propiophenon
2-[2'-Hydroxy-3'-(2-hydroxyethylamino)-propoxy]-β-(3-thienyl)-propiophenon
2-[2'-Hydroxy-3'-(2,2-dimethylpropylamino)-propoxy]-β-(3-thienyl)-propiophenon
2-(2'-Hydroxy-3'-n-propylamino-propoxy)-β-(2-pyridyl)-propiophenon
2-(2'-Hydroxy-3'-n-propylamino-propoxy)-β-(4-pyridyl)-propiophenon
2-(2'-Hydroxy-3'-tert.-butylamino-propoxy)-β-(2-furyl)-propiophenon
2-[2'-Hydroxy-3'-(2-(3,4-dimethoxyphenyl)-ethylamino)-propoxy]-β-(2-furyl)-propiophenon
2-[2'-Hydroxy-3'-(2-dimethylaminoethylamino)-propoxy]-β-[2-(1-methyl-pyrryl)]-propiophenon
2-[2'-Hydroxy-3'-(4-methyl-1-piperazinyl)-propoxy]-β-[2-(1-methyl-pyrryl)]-propiophenon
2-(2'-Hydroxy-3'-piperidino-propoxy)-β-[2-(1-methyl-pyrryl)]-propiophenon
2-[2'-Hydroxy-3'-(1-butin-3-yl-amino)-propoxy]-β-[2-(1-methyl-pyrryl)]-propiophenon
2-[2'-Hydroxy-3'-[4-(2-methoxyphenyl)-1-piperazinyl]-propoxy-β-[3-(1-methyl-pyrryl)]-propiophenon
2-(2'-Hydroxy-3'-morpholino-propoxy)-β-[4-(1-methyl-pyrazolyl)]-propiophenon
2-[2'-Hydroxy-3'-(4-methyl-1-piperazinyl)-propoxy]-β-[4-(1-methyl-pyrazolyl)]-propiophenon
2-(2'-Hydroxy-3'-allylamino-propoxy)-β-[4-(1-methyl-pyrazolyl)]-propiophenon

2-[2'-Hydroxy-3'-(3-dimethylaminopropylamino)-propoxy]-β-[4-(1-methyl-pyrazolyl)]-propiophenon
2-[2'-Hydroxy-3'-(4-hydroxy-4-phenyl-piperidino)-propoxy]-β-]4-(1-methyl-pyrazolyl)]-propiophenon
2-(2'-Hydroxy-3'-amino-propoxy)-β-[4-(1-methyl-pyrazolyl]-propiophenon
2-(2'-Hydroxy-3'-methylamino-propoxy)-β-[4-(1-methyl-pyrazolyl)]-propiophenon
2-(2'-Hydroxy-3'-cyclohexylamino-propoxy)-β-[4-(1-methyl-pyrazolyl)]-propiophenon
2-[2'-Hydroxy-3'-(2-(3,4-dimethoxyphenyl)-ethylamino)-propoxy]-β-[4-(1-methyl-pyrazolyl)]-propiophenon

Die erfindungsgemäßen Verbindungen können hergestellt werden, indem man

a) eine Verbindung der Formel

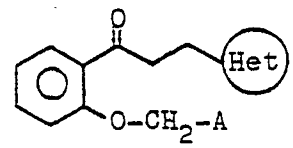

(II)

in der A den Rest

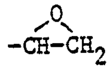

oder

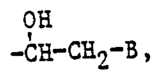

wobei B für eine nucleofuge Abgangsgruppe steht, bedeutet, mit einem Amin der Formel

$$HNR^1R^2 \qquad III,$$

in der $R^1$ und $R^2$ die angegebenen Bedeutungen haben, umsetzt oder

b) eine Verbindung der Formel IV

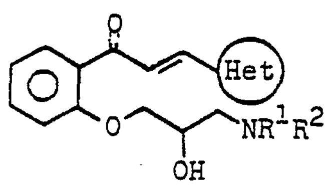

(IV)

worin $R^1$ und $R^2$ die angegebene Bedeutung besitzen, katalytisch hydriert, und gegebenenfalls die so erhaltenen Verbindungen in ihre Säureadditionssalze physiologisch verträglicher Säuren überführt.

Im Falle des Verfahrens a) stellt die Abgangsgruppe B bevorzugt ein Halogenatom, insbesondere ein Chlor-, Brom- oder Iodatom dar. Weiterhin kommen beispielsweise als nucleofuge Abgangsgruppen aromatische oder aliphatische Sulfonsäurereste in Betracht, wie der p-Toluolsulfonsäure- oder der Methansulfonsäurerest.

Die Umsetzungen werden bei Raumtemperaturen oder bei höheren Temperaturen, zweckmäßig bei Temperaturen von 50 bis 120 °C durchgeführt. Die Umsetzungen können unter Atmosphärendruck oder in einem geschlossen Gefäß unter erhöhtem Druck gegebenenfalls unter Erwärmung auf den angegebenen Temperaturbereich durchgeführt werden.

Die Ausgangsverbindungen können direkt, d. h. ohne Zugabe eines Verdünnungs- oder Lösungsmittels, umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Ethanol oder Propanol, vorzugsweise Isopropanol oder Ethanol, eines niederen gesättigten Dialkylethers, Dialkylglykolethers oder cyclischen Ethers, wie Diethylether, 1,2-Dimethoxyethan, Tetrahydrofuran oder Dioxan, Benzol oder eines Alkylbenzols, wie Toluol oder Xylol, oder eines aliphatischen Kohlenwasserstoffs, wie Hexan, Heptan oder Octan, eines niederen aliphatischen Ketons, wie Aceton, Methylethylketon oder Methyl-isobutylketon, eines Dialkylformamids, wie Dimethyl- oder Diethylformamid, von Dimethylsulfoxid oder in Gegenwart von Wasser oder in Mischungen der genannten Lösungsmittel, durchgeführt. Auch ist das in überschüssiger Menge verwendete Amin der allgemeinen Formel $HNR^1R^2$ gegebenenfalls als Verdünnungs- oder Lösungsmittel geeignet.

Bevorzugte Lösungsmittel bei der Umsetzung der Verbindungen II

(R= 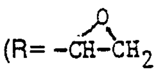 )

mit einem Amin $HNR^1R^2$ sind niedere Alkohole, insbesondere Ethanol oder Isopropanol, wobei die Umsetzung bevorzugt bei Temperaturen von 50 bis 120 °C und bei Normaldruck durchgeführt wird.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindungen erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der allgemeinen Formel II sind zum Teil bekannt oder können wie folgt hergestellt werden :

o-Hydroxyacetophenon wird mit einem heterocyclischen Aldehyd zu den α, β-ungesättigten Ketonen nach literaturbekannten Methoden kondensiert, wie sie beispielsweise in Org. Reactions Band 16, S. 1ff, John Wiley Verlag, New York, 1968, in Houben-Weyl, Methoden der organischen Chemie, Band 7/2b, S 1457 ff. G. Thieme Verlag, Stuttgart, 1976 oder in Chem. Ber. *94*, 26 (1961) beschrieben sind. Diese Ketone werden zu den entsprechenden β-substituierten 2-Hydroxy-propiophenonen ebenfalls nach literaturbekannten Methoden katalytisch hydriert, wie es beispielsweise in R.N. Rylander, « Catalytic Hydrogenation over Pt-Metals », S. 282, Academic Press 1967, beschrieben ist.

Die Überführung dieser β-substituierten 2-Hydroxy-propiophenone in die Propiophenone der Formel II erfolgt durch Alkylierung mit einem Epihalogenhydrin oder einem 1,3-Dihalogen-2-propanol nach an sich bekannten Verfahren.

Als Epihalogenhydrine kommen Epichlorhydrin, Epibromhydrin oder Epiiodhydrin und als 1,3-Dihalogen-2-propanole kommen insbesondere 1,3-Dichlor-2-propanol und 1,3-Dibrom-2-propanol in Betracht.

Die Umsetzungen der β-substituierten 2-Hydroxy-propiophenone zur Herstellung der Verbindungen der Formel II werden bei Temperaturen von 50 bis 80 °C und unter Normaldruck oder unter erhöhten Drucken in einem inerten Verdünnungs- oder Lösungsmittel, z. B. Aceton, Methanol oder Dimethylformamid in Gegenwart einer Base wie Kaliumcarbonat als säurebindendem Mittel vorgenommen.

Die β-substituierten 2-Hydroxy-propiophenone und die Ausgangsverbindungen II können zum Teil ohne vorherige Reinigung direkt in die nachfolgenden Reaktionsschritte eingesetzt werden.

Das Verfahren b) gelingt gut in alkoholischer Lösung. Als Katalysatoren eignen sich insbesondere Edelmetallkatalysatoren, wie Palladium, auf Kohle.

Die erfindungsgemäßen Verbindungen der Formel I besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Brom-campher-8-sulfonsäure, in die optisch aktiven Antipoden getrennt werden können.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht : Salzsäure, Bromwasserstoffsäure, Phosphorsäure oder Schwefelsäure, und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band 10, Seiten 224 bis 225, Birkhäuser Verlag, Basel und Stuttgart, 1966 entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanolderivate der Formel I durch Auflösen der freien Basen in einer wäßrigen Säurelösung hergestellt werden.

Die erfindungsgemäßen Verbindungen und ihre physiologisch verträglichen Säureadditionssalze sind wegen ihrer antiarrhythmischen, β-sympatholytischen und Ca-antagonistischen Eigenschaften insbesondere zur Pharmakotherapie von Herzrhythmusstörungen und zur Prophylaxe des plötzlichen Herztodes sowie zur Behandlung der coronaren Herzkrankheiten geeignet. Sie übertreffen den Wirkstoff Propafenon hinsichtlich Wirkstärke nach oraler Applikation deutlich.

Gegenstand der vorliegenden Erfindung sind demnach auch therapeutische Mittel oder Zubereitungen, die neben pharmazeutisch üblichen Träger- und Verdünnungsmitteln eine Verbindung der Formel I oder eines ihrer physiologisch verträglichen Säureadditionssalze als Wirkstoff enthalten, sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt (vgl. R. Voigt, Lehrbuch der pharmazeutischen Technologie, VEB-Verlag Volk und Gesundheit, Berlin 1975). Als therapeutische Einzeldosen kommen Dosierungen von 1 bis 500 mg, bevorzugt 5 bis 100 mg, in Betracht.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt. Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Saccharin, Cyclamat oder Zucker sowie z. B. Aromastoffe, wie Vanillin oder Orangenextrakt, enthalten. Sie können außerdem Suspendierhilfsstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln, wie Neutralfetten oder Polyethylenglykol bzw. deren Derivate herstellen.

Die folgenden Beispiele sollen die Erfindung näher erläutern.

A) Herstellung der Ausgangsverbindungen

Beispiel I

3-Oxo-1-(2-thienyl)-3-(2'-hydroxy-phenyl)-propen

Zu einer Mischung aus 150 Ethanol und 20 g NaOH in 100 ml $H_2O$ wurden bei Raumtemperatur 20,4 g o-Hydroxy-acetophenon und 16,8 g Thiopen-2-aldehyd gegeben. Die Reaktionsmischung wurde unter gutem Rühren 60 min auf 50 °C gebracht ; nach dem Abkühlen wurde mit 2n HCl unter gutem Kühlen neutralisiert. Der entstandene Niederschlag wurde abgesaugt, mit $H_2O$ gut gewaschen und anschließend aus Aceton/$H_2O$ umkristallisiert. Es wurden 24,5 g (= 71 % Ausbeute) des gelben Kristallisats vom Fp. 98 °C erhalten.

Analog wurden hergestellt :

3-Oxo-1-(2-furyl)-3-(2'-hydroxy-phenyl)-propen, Fp. 106 °C ;
3-Oxo-1-(3-pyridyl)-3-(2'-hydroxy-phenyl)-propen, Fp. 152 °C ;
3-Oxo-1-[2-(1-methyl-pyrryl)]-3-(2'-hydroxy-phenyl)-propen, Fp. 80-84 °C ;
3-Oxo-1-[3-(1-methyl-pyrryl)]-3-(2'-hydroxy-phenyl)-propen, Fp. 117-120 °C ;
3-Oxo-1-[4-(1-methyl-pyrazolyl)]-3-(2'-hydroxy-phenyl)-propen, Fp. 152 °C.

Beispiel II

2-Hydroxy-β-(2-thienyl)-propiophenon

8 g 3-Oxo-1-(2-thienyl)-3-(2'-hydroxy-phenyl)-propen wurden in 200 ml Methanol gelöst und in Gegenwart von Raney-Nickel-Kontakt bei 40 bis 50 °C unter Normaldruck hydriert. Nach ca. 4 h war die Wasserstoffaufnahme beendet. Nach dem Abkühlen wurde vom Katalysator abfiltriert und das Lösungsmittel unter vermindertem Druck abdestilliert. Es wurden 7,6 g (= 95 % Ausbeute) eines farblosen, öligen Rückstands isoliert, der bei längerem Stehen durchkristallisiert (Fp. 38 bis 42 °C).

Analog wurden hergestellt :

2-Hydroxy-β-(2-furyl)-propiophenon, ölig ;
2-Hydroxy-β-(3-pyridyl)-propiophenon, Fp. 68-73 °C ;
2-Hydroxy-β-[2-(1-methyl-pyrryl)]-propiophenon, ölig ;
2-Hydroxy-β-[3-(1-methyl-pyrryl)]-propiophenon, Fp. 44-45 °C ;
2-Hydroxy-β-[4-(1-methyl-pyrazolyl)]-propiophenon, Fp. 74-79 °C.

## Beispiel III

2-(2',3'-Epoxypropoxy)-β-(2-thienyl)-propiophenon

8,2 g 2-Hydroxy-β-(2-thienyl)-propiophenon wurden mit 15,5 g Epichlorhydrin in Gegenwart von 10,7 g wasserfreiem $K_2CO_3$ in 25 ml DMF 20 h lang bei 80 °C unter gutem Rühren gehalten. Nach dem Abkühlen wurde mit 150 ml $H_2O$ versetzt, mehrere Male mit Ether extrahiert, die vereinigten Etherextrakte mit $Na_2SO_4$ getrocknet und unter vermindertem Druck das Lösungsmittel und überschüssiges Epichlorhydrin abdestilliert. Es verblieben 9,2 g (= 91,5 % Ausbeute) öliger Rückstand, der ohne weitere Reinigung eingesetzt wurde.

Analog wurden alle übrigen Glycidether hergestellt und ohne weitere Reinigung für die nachfolgende Umsetzung verwendet.

## Beispiel IV

2-[2'-Hydroxy-3'-(N-isopropyl-N-benzyl)-amino]-propoxy-acetophenon-hydrochlorid

20 g 2-(2',3'-Epoxy-propoxy)-acetophenon wurden in 150 ml Isopropanol gelöst und mit 16 ml N-Isopropyl-N-benzyl-amin versetzt. Die Mischung wurde 8 h lang zum Rückfluß erhitzt. Nach dem Abkühlen wurde das Lösungsmittel unter vermindertem Druck abdestilliert und der verbleibende Rückstand mit etherischer Salzsäure in das Hydrochlorid überführt. Nach Umkristallisation aus Aceton/Methanol/Ether wurden 25,1 g (= 64 % Ausbeute) vom Fp. 143-146 °C erhalten.

## Beispiel V

3-Oxo-1-(3-pyridyl)-3-[2-(2'-hydroxy-3'-(N-isopropyl-N-benzyl-amino)-propoxy)-phenyl]-propen

10 g 2-[2'-Hydroxy-3'-(N-isopropyl-N-benzyl)-amino]-propoxy-acetophenon wurden in 300 ml Methanol gelöst und mit 3,3 g Pyridin-3-aldehyd in Gegenwart von 4 g NaOH durch 5- bis 6stündiges Erwärmen auf 45 °C kondensiert. Nach dem Abkühlen wurde mit 500 ml $H_2O$ versetzt und so 8 g gelbes Kristallisat (= 64 % Ausbeute) vom Fp. 145 °C erhalten.

## B) Herstellung der erfindungsgemäßen Verbindungen

## Beispiel 1

2-(2'-Hydroxy-3'-n-propyl-amino-propoxy)-β-(2-thienyl)-propiophenon-hydrochlorid

8 g 2-(2',3'-Epoxy-propoxy)-β-(2-thienyl)-propiophenon und 9,5 g n-Propyl-amin werden in 150 ml Isopropanol gelöst und 8 h lang zum Rückfluß erhitzt. Nach dem Erkalten wurde das Lösungsmittel unter vermindertem Druck abdestilliert ; es verblieben 10,3 g öliger Rückstand, der nach längerem Stehen kristallisierte. Mit Hilfe von etherischer Salzsäure wurden, nach Umkristallisation aus Aceton/Methanol/Ether, 3,5 g (= 35 % Ausbeute) des Hydrochlorids vom Fp. 163 °C isoliert.

Analog Beispiel 1 wurden aus den entsprechenden Ausgangsmaterialien folgende Verbindungen hergestellt :

| Bsp. Nr. | Het | $NR^1R^2$ | Fp. [°C] | Salz | M.G. | Summenformel |
|---|---|---|---|---|---|---|
| 2 | 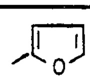 | NH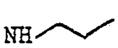 | 150–155 | Hydrochlorid | 367,5 | $C_{19}H_{26}ClNO_4$ |
| 3 | 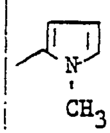 | NH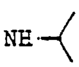 | 103 | freie Base | 344 | $C_{20}H_{28}N_2O_3$ |
| 4 | " | NH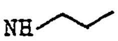 | 137–138 | Fumarat | 461 | $C_{24}H_{32}N_2O_7$ |

(Suite)

| Bsp. Nr. | Het | $NR^1R^2$ | Fp. [°C] | Salz | M.G. | Summenformel |
|---|---|---|---|---|---|---|
| 5 | " | 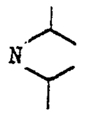 | 118 | 1/2 Fumarat | 444 | $C_{25}H_{36}N_2O_5$ |
| 6 | 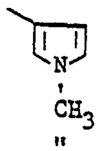 | 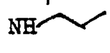 | 132 | Hydrochlorid | 380,5 | $C_{20}H_{29}ClN_2O_3$ |
| 7 | " | 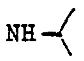 | 151-153 | 1/2 Fumarat | 402 | $C_{22}H_{30}N_2O_5$ |
| 8 | " | 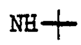 | 150-153 | 1/2 Fumarat | 416 | $C_{23}H_{32}N_2O_5$ |
| 9 | 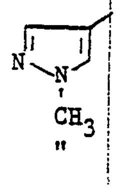 | 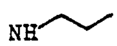 | 122 | Hydrochlorid | 381,5 | $C_{19}H_{28}ClN_3O_3$ |
| 10 | " | 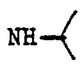 | 122-125 | Hydrochlorid | 381,5 | $C_{19}H_{28}ClN_3O_3$ |
| 11 | " | 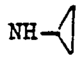 | 140-143 | Oxalat | 433 | $C_{21}H_{27}N_3O_7$ |
| 12 | " | 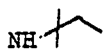 | 131 | Fumarat | 489 | $C_{25}H_{35}N_3O_7$ |
| 13 | " | 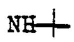 | 185-188 | Hydrochlorid | 395,5 | $C_{20}H_{30}ClN_3O_3$ |
| 14 | " | 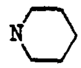 | 122-125 | Hydrochlorid | 408,5 | $C_{21}H_{30}ClN_3O_3$ |
| 15 | " | 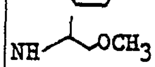 | 72- 76 | Fumarat | 491 | $C_{24}H_{33}N_3O_8$ |

## Beispiel 16

2-(2'-Hydroxy-3'-iso-propylamino-propoxy)-β-(3-pyridyl-propiophenon)-oxalat

8 g 3-Oxo-1-(3-pyridyl)-3-[2-(2'-hydroxy-3'-(N-isopropyl-N-benzyl-amino)-propoxy)-phenyl]-propen wurden in Essigester (150 ml) gelöst und in Gegenwart von Raney-Nickel unter Normaldruck bei 40 bis 50 °C bis zur Beendigung der Wasserstoffaufnahme (320 $cm^3$) hydriert. Nach dem Abfiltrieren des Katalysators wurde das Lösungsmittel unter vermindertem Druck abdestilliert. Es verblieben 8,1 g des farblosen, öligen 2-[2'-Hydroxy-3'-(N-isopropyl-N-benzyl-amino)-propoxy]-β-(3-pyridyl)-propiophenons, welches ohne weitere Reinigung in 200 ml Ethanol in Gegenwart von 0,8 g Pd-C-Katalysator (10 %ig) unter Normaldruck bei 40 bis 50 °C bis zur Beendigung der $H_2$-Aufnahme hydriert wurde. Nach Abfiltrieren des Katalysators wurde das Lösungsmittel unter vermindertem Druck abdestilliert. Der verbleibende Rückstand wurde in Aceton gelöst und mit Oxalsäure (gelöst in Ethanol) versetzt. Nach Zugabe von Ether wurden 2,5 g (= 26 % Ausbeute) Kristallisat vom Fp. 92-96 °C isoliert.

## C) Anwendung

Die pharmakologischen Untersuchungen zur Bestimmung der antiarrhythmischen Wirkung wurden im Vergleich zu den bekannten Antiarrhythmica Propafenon 2-(2'-Hydroxy-3'-n-propylamino-propoxy)-ω-phenyl-propiophenon (DE-OS 20 01 431) und Diprafenon 2-[2-Hydroxy-3-(1,1-dimethylpropylamino)-propoxy]-ω-phenyl-propiophenon DE-OS 31 33 814) an Hunden durchgeführt, weil Propafenon an dieser Tierspezies ähnlich wie beim Menschen metabolisiert wird (vgl. HEGE, H.G. et al., European Journal of Drug Metabolism and Pharmacokinetics, *9*, 41 (1984) und HEGE, H.G. et al., Arzneimittelforschung/Drug Research *9*, 34 (1984).

Verwendet wurden männliche oder weibliche Bastardhunde im Gewicht von 12-15 kg. Die Applikation der zu prüfenden Substanzen erfolgte per os, 40 min vor Einleitung einer Pentobarbital-Na-Narkose (30 mg/kg. i.v.). Herzrhythmusstörungen wurden durch Infusion von Aconitin (5 µg/kg · min) ausgelöst. Die Infusion begann 60 min nach Substanzapplikation. Bei unbehandelten Tieren traten im EKG Rhythmusstörungen (Verlust oder Umkehr von P, ventrikuläre Tachyarrhythmien) nach durchschnittlich 6,4 ± 0,29 min Infusionsdauer auf.

Wenn man die erfindungsgemäße Substanzen in einer Dosis von 10 mg/kg appliziert, wird die Zeit bis

zum Auftreten der Arrhythmien verlängert. Im Vergleich zu Propafenon und dem gleichwertigen Diprafenon ist diese Verlängerung zwei- (Beispiel 4) bis sechsmal größer (Beispiel 7) ; die antiarrhythmische Wirksamkeit ist höher (Tabelle 1).

Tabelle 1

Antiarrhythmische Wirkung an der Aconitinarrhythmie des Hundes (10 mg/kg, Applikation per os)

| Beispiel Nr. | Verlängerung der Aconitininfusionsdauer (Δ %) |
|---|---|
| 9 | 154 |
| 3 | -85 |
| 12 | 137 |
| 4 | 70 |
| 5 | 85 |
| 7 | 193 |
| Propafenon | 34 |
| Diprafenon | 33 |

Weiterhin wurden die Substanzen hinsichtlich ihrer Wirkung auf Arrhythmien nach Coronarligatur geprüft. Diese Prüfmethode läßt Rückschlüsse auf die Wirksamkeit auf Infarktarrhythmien beim Menschen zu. Es wurden männliche und weibliche Beagle-Hunden im Körpergewicht von 9-15 kg verwendet. Der Ramus descendens der linken Coronararterie wurde nach einer modifizierten Methode von Harris (Circulation *1*, 1318-1328, 1950) unterbunden. Hierdurch kommt es zu elektrocardiographis nachweisbaren tachykarden, meist ventrikulären Arrhythmien verschiedenen Ursprungs mit P-Verlust, QRS-Verformung und T-Wellen-Erhöhung. Als Meßgröße für die Bestimmung der antiarrhythmischen Wirkung diente die Häufigkeit (%) der normalen Herzaktionen, die während einer Kontrollperiode von 100 Minuten im Abstand von 5 Minuten ermittelt wurden. Als Voraussetzung für eine Substanzapplikation durften während dieser Kontrollperiode nicht mehr als 29 % normale Herzschläge auftreten. Nach Substanzapplikation wurde das EKG zunächst wie in der Kontrollperiode 100 Minuten lang im Abstand von 5 Minuten und anschließend für weitere 200 Minuten im Abstand von 10 Minuten ausgewertet.

Wie aus der Tabelle 2 hervorgeht, sind die erfindungsgemäßen Substanzen auch an diesem Versuchsmodell wirksamer als Propafenon und Diprafenon. So hemmt Beispiel 9 bereits in einer ca. 5 mal niedrigeren oralen Dosis die Arrhythmien ebenso stark wie Propafenon und deutlich stärker als Diprafenon. Die Beispiele 5 und 7 sind mit 10 mg/kg aktiver als Propafenon und Diprafenon in der gleichen Dosis.

Tabelle 2

Antiarrhythmische Wirkung bei Infarktarrhythmien am wachen Hund

| Beispiel Nr. | Zunahme der normalen Herzaktionen %<br>Dosis mg/kg<br>4.64 | <br><br>10.0 | <br><br>21.5 |
|---|---|---|---|
| 5 | - | 66 | - |
| 7 | - | 92 | - |
| 9 | 74 | - | - |
| Propafenon | 5 | 40 | 70 |
| Diprafenon | - | - | 43 |

An weiblichen NMRI-Mäusen im Gewicht von 24-28 g durchgeführte Toxizitätsbestimmungen (Applikation i.p.) zeigen, daß die letalen Dosen der erfindungsgemäßen Substanzen in der gleichen Größenordnung liegen wie die der Vergleichssubstanzen. Daraus ergibt sich, daß die Zunahme der Wirksamkeit nicht mit einer entsprechenden Toxizitätssteigerung einhergeht (Tabelle 3).

Tabelle 3

Letale Dosen nach einmaliger Applikation (i.p.) an Mäusen

| Beispiel | LD 50 (mg/kg [a]) |
|---|---|
| 9 | 31,6 |
| 3 | 68,1 |
| 12 | 68,1 |
| 4 | 46,4 |
| 5 | 68,1 |
| 7 | 46,4 |
| Propafenon | 46,4 |
| Diprafenon | 68,1 |

[a] Näherungswerte

**Patentansprüche** (für die Vertragsstaaten : BE, CH, DE, FR, GB, IT, LI, NL, SE)

1. Aminopropanolderivate der Formel

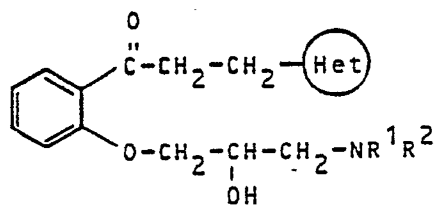

(I)

in der

$R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu insgesamt 9 C-Atomen, oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5-bis 7-gliedrigen gesättigten heterocyclischen Ring bilden, der durch einen oder zwei Phenyl- und/oder Hydroxyreste substituiert sein und ein Sauerstoff- oder Stickstoffatom als weiteres Heteroatom im Ring enthalten kann, wobei ein solches zusätzliches Stickstoffatom durch einen Alkylrest mit 1 bis 3 C-Atomen oder einen Phenylrest substituiert sein kann, und

—(Het) die Reste 2-Thienyl, 3-Thienyl, 2-Pyridyl, 3-Pyridyl, 4-Pyridyl, 2-Furyl, 2-(1-Alkyl)-pyrryl, 3-(1-Alkyl)-pyrryl und 4-(1-Alkyl)-pyrazolyl bedeutet, wobei der Alkylrest 1 bis 13 Kohlenstoffatome enthält, und ihre physiologisch verträglichen Säureadditionssalze.

2. Aminopropanolderivate der Formel I nach Anspruch 1, dadurch gekennzeichnet, daß die Gruppe $NR^1R^2$ den Piperidin-, Piperazin-, N-Methylpiperazin-, Morpholin- oder Diisopropylaminorest oder $R^1$ oder $R^2$ oder beide Wasserstoff, n-Propyl, Isopropyl, n-Butyl, Isobutyl, tert.-Butyl, n-Pentyl, sec.-Pentyl, Isopentyl, Neopentyl, β-Methoxyethyl oder β-Hydroxyethyl darstellen.

3. Aminopropanolderivate der Formel I nach Anspruch 1, dadurch gekennzeichnet, daß die Gruppe (Het) Pyridyl, 2-(1-Methyl)-pyrryl, 3-(1-Methyl)-pyrryl oder 4-(1-Methyl)-pyrazolyl darstellt.

4. Verfahren zur Herstellung von Aminopropanolderivaten der Formel I nach Anspruch 1 und deren physiologisch verträglichen Salzen, dadurch gekennzeichnet, daß man

a) eine Verbindung der Formel II

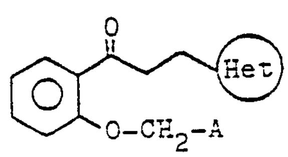

(II)

in der A den Rest

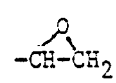

oder

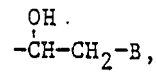

wobei B für eine nucleofuge Abgangsgruppe steht, bedeutet, mit einem Amin der Formel

$$HNR^1R^2 \qquad III,$$

in der $R^1$ und $R^2$ die angegebenen Bedeutungen haben, umsetzt oder

b) eine Verbindung der Formel IV

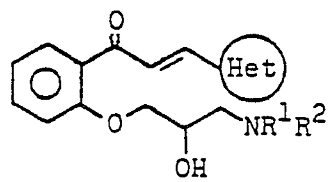

(IV)

worin $R^1$ und $R^2$ die angegebene Bedeutung besitzen, katalytisch hydriert, und gegebenenfalls die so erhaltenen Verbindungen in ihre Säureadditionssalze physiologisch verträglicher Säuren überführt.

5. Antiarrhythmikum, das neben den üblichen galenischen Hilfs- und Verdünnungsmitteln als Wirkstoff eine wirksame Menge einer Verbindung der Formel I nach Anspruch 1 enthält.

6. Aminopropanolderivate der Formel I nach Anspruch 1 oder ein Antiarrhythmikum nach Anspruch 5 zur Anwendung in der Therapie von Herzrhythmusstörungen und in der Prophylaxe des plötzlichen Herztodes sowie in der Behandlung der coronaren Herzkrankheiten.

**Patentansprüche** (für die Vertragsstaaten AT)

1. Verfahren zur Herstellung von Aminopropanolderivaten der Formel I

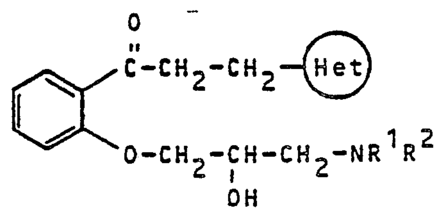

(I)

in der

$R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu insgesamt 9 C-Atomen, oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5-bis 7-gliedrigen gesättigten heterocyclischen Ring bilden, der durch einen oder zwei Phenyl- und/oder Hydroxyreste substituiert sein und ein Sauerstoff- oder Stickstoffatom als weiteres Heteroatom im Ring enthalten kann, wobei ein solches zusätzliches Stickstoffatom durch einen Alkylrest mit 1 bis 3 C-Atomen oder einen Phenylrest substituiert sein kann, und

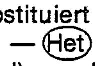 die Reste 2-Thienyl, 3-Thienyl, 2-Pyridyl, 3-Pyridyl, 4-Pyridyl, 2-Furyl, 2-(1-Alkyl)-pyrryl, 3-(1-Alkyl)-pyrryl und 4-(1-Alkyl)-pyrazolyl bedeutet, wobei der Alkylrest 1 bis 3 Kohlenstoffatome enthält, und deren physiologisch verträglichen Salzen, dadurch gekennzeichnet, daß man

a) eine Verbindung der Formel II

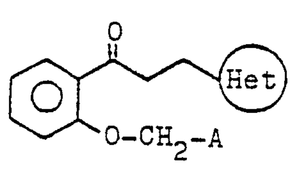

(II)

in der A den Rest

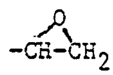

oder

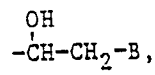

wobei B für eine nucleofuge Abgangsgruppe steht, bedeutet, mit einem Amin der Formel

$$HNR^1R^2 \qquad III,$$

in der $R^1$ und $R^2$ die angegebenen Bedeutungen haben, umsetzt oder

b) eine Verbindung der Formel IV

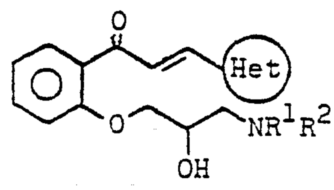

(IV)

worin $R^1$ und $R^2$ die angegebene Bedeutung besitzen, katalytisch hydriert, und gegebenenfalls die so erhaltenen Verbindungen in ihre Säureadditionssalze physiologisch verträglicher Säuren überführt.

2. Verfahren zur Herstellung von Aminopropanolderivaten der Formel I nach Anspruch 1, wobei die Gruppe $NR^1R^2$ den Piperidin-, Piperazin-, N-Methyl-piperazin-, Morpholin- oder Diisopropylaminorest oder $R^1$ oder $R^2$ oder beide Wasserstoff, n-Propyl, Isopropyl, n-Butyl, Isobutyl, tert.-Butyl, n-Pentyl, sec.-Pentyl, Isopentyl, Neopentyl, β-Methoxyethyl oder β-Hydroxyethyl darstellen.

3. Verfahren zur Herstellung von Aminopropanolderivaten der Formel I nach Anspruch 1, wobei die Gruppe (Het) Pyridyl, 2-(1-Methyl)-pyrryl, 3-(1-Methyl)-pyrryl oder 4-(1-Methyl)-pyrazolyl darstellt.

**Claims** (for the Contracting states : BE, CH, DE, FR, GB, IT, LI, NL, SE)

1. An aminopropanol derivative of the formula

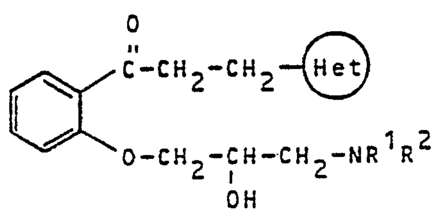

(I)

where

$R^1$ and $R^2$ are identical or different and are each hydrogen, alkyl, cycloalkyl, alkenyl, alkynyl or hydroxyalkyl, each of up to 6 carbon atoms, alkoxyalkyl, alkylthioalkyl or dialkylaminoalkyl, each of up to a total of 9 carbon atoms, or phenylalkyl or phenoxyalkyl, where the alkyl radical is of up to 6 carbon atoms and the phenyl radical is unsubstituted or substituted by alkyl or alkoxy, each of up to 3 carbon atoms, or

$R^1$ and $R^2$, together with the nitrogen atom which links them, form a 5-membered to 7-membered saturated heterocyclic ring which can be substituted by one or two phenyl and/or hydroxyl radicals and can contain an oxygen or nitrogen atom, as a further heteroatom in the ring, and such an additional nitrogen atom can be substituted by alkyl of 1 to 3 carbon atoms or by phenyl, and

—(Het) is thien-2-yl, thien-3-yl, pyrid-2-yl, pyrid-3-yl, pyrid-4-yl, fur-2-yl, 1-alkylpyrr-2-yl, 1-alkylpyrr-3-yl or 1-alkylpyrazol-4-yl, alkyl being of 1 to 3 carbon atoms,

and its physiologically tolerated addition salts with acids.

2. Am aminopropanol derivative of the formula I as claimed in claim 1, wherein the group $NR^1R^2$ is a piperidine, piperazine, N-methylpiperazine, morpholine or diisopropylamino radical, or $R^1$ or $R^2$ is, or $R^1$ and $R^2$ are, hydrogen, n-propyl, isopropyl, n-butyl, isobutyl, tert.-butyl, n-pentyl, sec.-pentyl, isopentyl,

neopentyl, β-methoxyethyl or β-hydroxyethyl.

3. An aminopropanol derivative of the formula I as claimed in claim 1, wherein the group (Het) is pyridyl, 1-methylpyrr-2-yl, 1-methylpyrr-3-yl or 1-methylpyrazol-4-yl.

4. A process for the preparation of an aminopropanol derivative of the formula I as claimed in claim 1 and its physiologically tolerated salts, wherein

a) a compound of the formula II

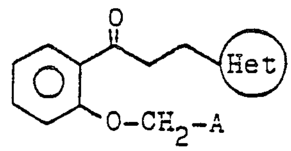

(II)

where A is

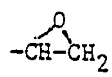

or

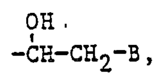

B being a nucleofugic leaving group, is reacted with an amine of the formula

$$HNR^1R^2 \qquad III,$$

where $R^1$ and $R^2$ have the stated meanings, or

b) a compound of the formula IV

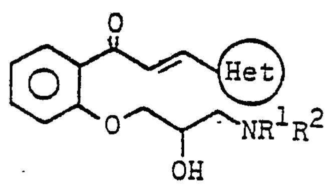

(IV)

where $R^1$ and $R^2$ have the stated meanings, is hydrogenated catalytically, and, if desired, the resulting compound is converted into its addition salts with physiologically tolerated acids.

5. An antiarrhythmic agent which contains, as the active compound, an effective amount of a compound of the formula I as claimed in claim 1, in addition to the conventional pharmaceutical auxiliaries and diluents.

6. An aminopropanol derivative of the formula I as claimed in claim 1, or an antiarrhythmic agent as claimed in claim 5 for use in the therapy of cardiac arrythmias, in the prophylaxis of sudden heart death, and in the treatment of coronary heart diseases.

**Claims** (for the Contracting state : AT)

1. A process for the preparation of an aminopropanol derivative of the formula I

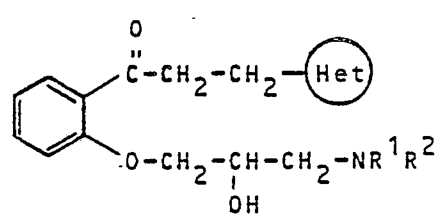

(I)

where

$R^1$ and $R^2$ are identical or different and are each hydrogen, alkyl, cycloalkyl, alkenyl, alkynyl or hydroxyalkyl, each of up to 6 carbon atoms, alkoxyalkyl, alkylthioalkyl or dialkylaminoalkyl, each of up to

a total of 9 carbon atoms, or phenylalkyl or phenoxyalkyl, where the alkyl radical is of up to 6 carbon atoms and the phenyl radical is unsubstituted or substituted by alkyl or alkoxy, each of up to 3 carbon atoms, or

$R^1$ and $R^2$, together with the nitrogen atom which links them, form a 5-membered to 7-membered saturated heterocyclic ring which can be substituted by one or two phenyl and/or hydroxyl radicals and can contain an oxygen or nitrogen atom, as a further heteroatom in the ring, and such an additional nitrogen atom can be substituted by alkyl of 1 to 3 carbon atoms or by phenyl, and

—Het is thien-2-yl, thien-3-yl, pyrid-2-yl, pyrid-3-yl, pyrid-4-yl, fur-2-yl, 1-alkylpyrr-2-yl, 1-alkylpyrr-3-yl or 1-alkylpyrazol-4-yl, alkyl being of 1 to 3 carbon atoms,

and its physiologically tolerated addition salts with acids, wherein

a) a compound of the formula II

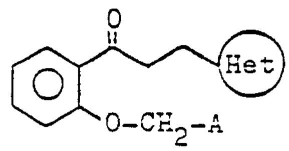

(II)

where A is

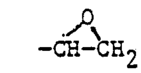

or

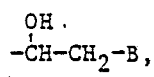

B being a nucleofugic leaving group, is reacted with an amine of the formula

$$HNR^1R^2 \qquad III,$$

where $R^1$ and $R^2$ have the stated meanings, or

b) a compound of the formula IV

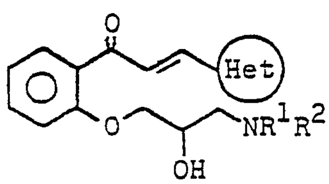

(IV)

where $R^1$ and $R^2$ have the stated meanings, is hydrogenated catalytically, and, if desired, the resulting compound is converted into its addition salts with physiologically tolerated acids.

2. A process for the preparation of an aminopropanol derivative of the formula I as claimed in claim 1, where the group $NR^1R^2$ is a piperidine, piperazine, N-methyl-piperazine, morpholine or diisopropylamino radical, or $R^1$ or $R^2$ is, or $R^1$ and $R^2$ are, hydrogen, n-propyl, isopropyl, n-butyl, isobutyl, tert.-butyl, n-pentyl, sec.-pentyl, isopentyl, neopentyl, β-methoxyethyl or β-hydroxyethyl.

3. A process for the preparation of an aminopropanol derivative of the formula I as claimed in claim 1, where the group Het is pyridyl, 1-methylpyrr-2-yl, 1-methylpyrr-3-yl or 1-methylpyrazol-4-yl.

**Revendications** (pour les Etats contractants : BE, CH, DE, FR, GB, IT, LI, NL, SE)

1. Dérivés d'aminopropanol de formule

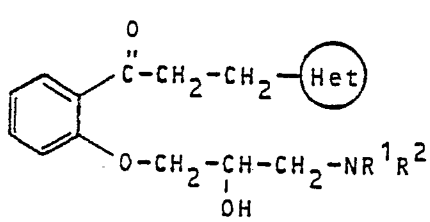

(I)

dans laquelle

$R^1$ et $R^2$ sont identiques ou différents et représentent des atomes d'hydrogène, des restes alkyle, cycloalkyle, alcényle, alcinyle ou hydroxyalkyle, ayant chacun jusqu'à 6 atomes C, des restes alcoxyalkyle, alkylthioalkyle ou dialkylaminoalkyle ayant chacun jusqu'à au total 9 atomes C, ou des restes phénylalkyle ou phénoxyalkyle ayant jusqu'à 6 atomes C dans l'alkyle et éventuellement substitués dans le reste phényle par alkyle ou alcoxy ayant chacun jusqu'à 3 atomes C, ou

$R^1$ et $R^2$ forment ensemble, avec l'atome d'azote qui les réunit, un noyau hétérocyclique saturé à 5 à 7 chaînons, qui peut être substitué par un ou deux restes phényle et/ou hydroxy et contenir dans le noyau, comme autre hétéroatome, un atome d'oxygène ou d'azote, un tel atome additionnel pouvant être substitué par un reste alkyle de 1 à 3 atomes C ou un reste phényle, et

— Het représente les restes 2-thiényle, 3-thiényle, 2-pyridyle, 3-pyridyle, 4-pyridyle, 2-furyle, 2-(1-alkyl)-pyrryle, 3-(1-alkyl)-pyrryle et 4-(1-alkyl)-pyrazolyle, le reste alkyle contenant 1 à 3 atomes de carbone,

et leurs sels d'addition d'acide acceptables physiologiquement.

2. Dérivés d'aminopropanol de formule I selon la revendication 1, caractérisés par le fait que le groupe $NR^1R^2$ représente le reste pipéridine, pipérazine, N-méthyl-pipérazine, morpholine ou diisopropylamino ou $R^1$ ou $R^2$, ou les deux, représentent hydrogène, n-propyle, isopropyle, isobutyle, tert-butyle, n-pentyle, sec-pentyle, isopentyle, néopentyle, β-méthoxyéthyl ou β-hydroxyéthyle.

3. Dérivés d'aminopropanol de formule I selon la revendication 1, caractérisés par le fait que le groupe Het représente pyridyle, 2-(1-méthyl)-pyrryle, 3-(1-méthyl)-pyrryle ou 4-(1-méthyl)-pyrazolyle.

4. Procédé de préparation de dérivés d'aminopropanol de formule I selon la revendication 1 et leurs sels acceptables physiologiquement, caractérisé par le fait que

a) on fait réagir un composé de formule II

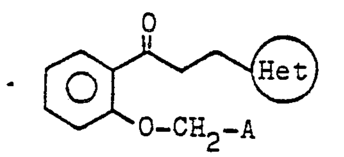

(II)

dans laquelle A représente le reste

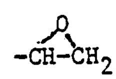

ou

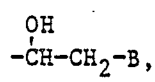

B étant mis pour un groupe éliminable nucléofuge, avec une amine de formule

$$HNR^1R^2 \qquad III$$

dans laquelle $R^1$ et $R^2$ ont les significations indiquées, ou

b) on hydrogène catalytiquement un composé de formule IV

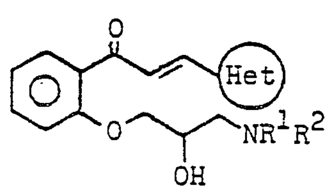

(IV)

où $R^1$ et $R^2$ ont la signification indiquée, et on transforme éventuellement les composés ainsi obtenus en leurs sels d'addition d'acides acceptables physiologiquement.

5. Antiarythmique qui, outre les diluants et additifs galéniques usuels, contient, comme principe actif, une quantité efficace d'un composé de formule I selon la revendication 1.

6. Dérivés d'aminopropanol de formule I selon la revendication 1 ou antiarythmique selon la revendication 5 pour utilisation dans la thérapeutique des troubles du rythme cardiaque et dans la prophylaxie de la mort par l'arrêt subit du cœur ainsi que dans le traitement des maladies cardiaques

coronariennes.

**Revendications** (pour l'Etat contractant : AT)

1. Procédé de préparation de dérivés d'aminopropanol de formule I

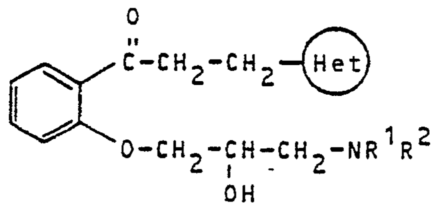

(I)

dans laquelle

$R^1$ et $R^2$ sont identiques ou différents et représentent des atomes d'hydrogène, des restes alkyle, cycloalkyle, alcényle, alcinyle ou hydroxyalkyle, ayant chacun jusqu'à 6 atomes C, des restes alcoxyalkyle, alkylthioalkyle ou dialkylaminoalkyle ayant chacun jusqu'à au total 9 atomes C, ou des restes phénylalkyle ou phénoxyalkyle ayant jusqu'à 6 atomes C dans l'alkyle et éventuellement substitués dans le reste phényle par alkyle ou alcoxy ayant chacun jusqu'à 3 atomes C, ou

$R^1$ et $R^2$ forment ensemble, avec l'atome d'azote qui les réunit, un noyau hétérocyclique saturé à 5 à 7 chaînons, qui peut être substitué par un ou deux restes phényle et/ou hydroxy et contenir dans le noyau, comme autre hétéroatome, un atome d'oxygène ou d'azote, un tel atome d'azote additionnel pouvant être substitué par un reste alkyle de 1 à 3 atomes C ou un reste phényle, et

— (Het) représente les restes 2-thiényle, 3-thiényle, 2-pyridyle, 3-pyridyle, 4-pyridyle, 2-furyle, 2-(1-alkyl)-pyrryle, 3-(1-alkyl)-pyrryle et 4-(1-alkyl)-pyrazolyle, le reste alkyle contenant 1 à 3 atomes de carbone,

et leurs sels d'addition d'acide acceptables physiologiquement, caractérisé par le fait que

a) on fait réagir un composé de formule II

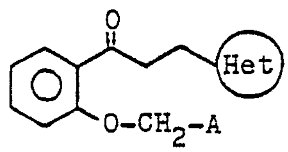

(II)

dans laquelle A représente le reste

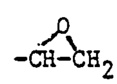

ou

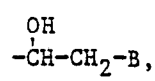

B étant mis pour un groupe éliminable nucléofuge, avec une amine de formule

$HNR^1R^2$ III,

dans laquelle $R^1$ et $R^2$ ont les significations indiquées, ou

b) on hydrogène catalytiquement un composé de formule IV

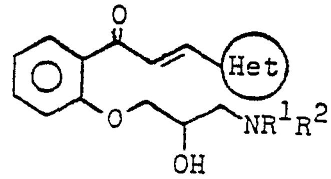

(IV)

où $R^1$ et $R^2$ ont la signification indiquée, et on transforme éventuellement les composés ainsi obtenus en leurs sels d'addition d'acides acceptables physiologiquement.

2. Procédé de préparation de dérivés d'aminopropanol de formule I selon la revendication 1, dans lequel le groupe $NR^1R^2$ représente le reste pipéridine, pipérazine, N-méthyl-pipérazine, morpholine ou diisopropylamino ou $R^1$ ou $R^2$,
ou les deux, représentent hydrogène, n-propyle, isopropyle, isobutyle, tert-butyle, n-pentyle, sec-pentyle, isopentyle, néopentyle, β-méthoxyéthyl ou β-hydroxyéthyle.

3. Procédé de préparation de dérivés d'aminopropanol de formule I selon la revendication 1, dans lequel le groupe Het représente pyridyle, 2-(1-méthyl)pyrryle, 3-(1-méthyl)-pyrryle ou 4-(1-méthyl)-pyrazolyle.